# EUROPÄISCHE PATENTSCHRIFT

(11) **EP 1 121 152 B1**
(45) Veröffentlichungstag und Bekanntmachung des Hinweises auf die Patenterteilung: **14.04.2004**
(21) Anmeldenummer: 99950719.7
(22) Anmeldetag: 13.10.1999
(51) Int. Cl.: A61K 47/48, A61K 31/57

(54) **KOMBINATION AUS NORPREGNAN DERIVATEN UND CYCLODEXTRIN**
COMBINATION OF NORPREGNANE DERIVATIVES AND CYCLODEXTRIN
COMBINAISON DE DERIVES DE NORREGNAN ET DE CYCLODEXTRINE

(30) Priorität: 14.10.1998 DE 19848303
(43) Veröffentlichungstag der Anmeldung: 08.08.2001
(73) Patentinhaber: SCHERING AKTIENGESELLSCHAFT, 13342 Berlin (DE)
(72) Erfinder: HÖFERT, Peter, D-12099 Berlin (DE); BACKENSFELD, Thomas, D-13127 Berlin (DE)
(86) Internationale Anmeldenummer: PCT/EP1999/007711
(87) Internationale Veröffentlichungsnummer: WO 2000/021570

(56) Entgegenhaltungen:
- EP-A- 0 349 091
- EP-A- 0 477 107
- WO-A-90/01320
- WO-A-96/02277
- WO-A-96/20209
- US-A- 4 383 992

## Beschreibung

Die Erfindung betrifft eine Kombination aus mindestens einem Gestagen und einem Zucker. Der Zucker stabilisiert das Gestagen dahingehend, daß die Acyloinumlagerung in der Seitenkette an den Atomen C₂₀ und C₂₁ sowie oxidative Zersetzung verhindert wird. Weiterhin umfaßt die Erfindung auch die Verwendung der Kombination als Arzneimittel und Verfahren zur Herstellung von Kombinationen.

### Stand der Technik

Aus der WO 96 / 02277 (Anmeldetag: 10. Juli 1996) sind Komplexe aus steroidalen Sexualhormonen und Cyclodextrin bekannt. Allein der Komplex aus 17α-Ethinylestradiol und β-Cyclodextrin wird konkret beschrieben.

In der Publikation WO 96/20209 mit Anmeldetag vom 4. Juli 1996 werden allgemein Gestagene beschrieben. Dabei wird insbesondere auch ein (21S)-21-Hydroxy-21-methyl-14,17-ethano-19-norpregna-4,9,15-triene-3,20-dion erwähnt. Gestagene werden zur Behandlung von klimakterischen Beschwerden eingesetzt. Auch läßt sich die Fertilität mit diesen Gestagenen kontrollieren.
Gestagene mit einer α-Hydroxyketonstruktur in der Seitenkette unterliegen bei Lagerung einer Acyloinumlagerung. Dabei treten sterische Varianten auf. Diese Umlagerung wird durch viele pharmazeutische Hilfsstoffe (z.B. Lactose, Magnesiumstearat) beschleunigt.
Darüber hinaus treten Oxidationsreaktionen in verschiedenen Molekülpositionen auf.

### Aufgabe und Lösung

Es stellt sich somit die Aufgabe, Gestagene, insbesondere (21S)-21-Hydroxy-21-methyl-14,17-ethano-19-norpregna-4,9,15-triene-3,20-dion gegen Zersetzung durch Acyloinumlagerung oder Oxidation zu schützen, ohne die pharmakologische Verträglichkeit und pharmazeutische Bearbeitung negativ zu beeinträchtigen.

Die Aufgabe wird gelöst durch eine Kombination aus mindestens einem Gestagen und einem β-Cyclodextrin oder γ-Cyclodextrin, oder Derivaten dieser Cyclodextrine, die durch Veretherung oder Veresterung freier alkoholischer Funktionen der Cyclodextrine erhalten werden,
wobei die Gestagene 14,17-C₂ - überbrückte Steroide sind, die zur Gruppe der Formel I zählen: 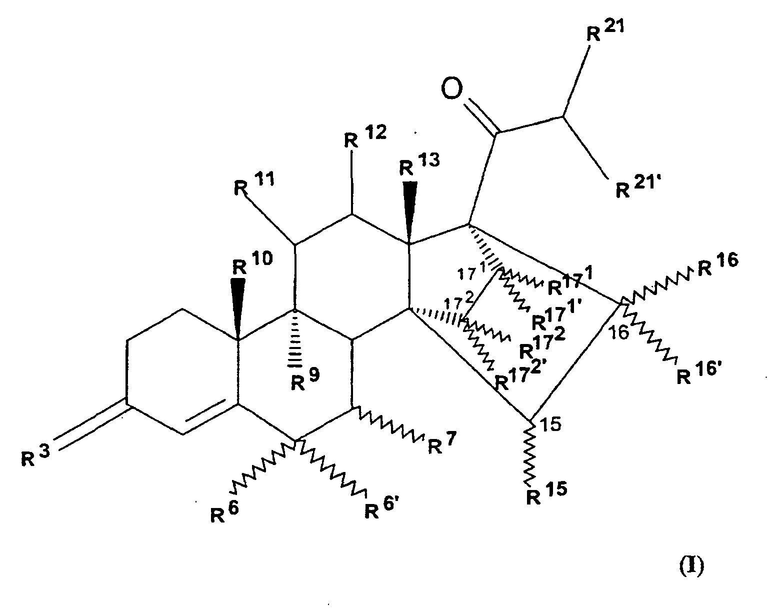 worin
- R³: für ein Sauerstoffatom, die Hydroxyiminogruppe oder zwei Wasserstoffatome,
- R⁶: für ein Wasserstoff-, Fluor-, Chlor- oder Bromatom oder für einen α- oder β-ständigen C₁-C₄-Alkylrest, wobei dann R⁶' und R⁷ Wasserstoffatome darstellen, oder aber
- R⁶': für ein Wasserstoff-, Fluor-, Chlor- oder Bromatom oder für einen C₁-C₄-Alkylrest, wobei dann R⁶' und R⁷ eine gemeinsame zusätzliche Bindung darstellen,
- R⁷: für einen α- oder β-ständigen C₁-C₄-Alkylrest, wobei dann R⁶ und R⁶' Wasserstoffatome darstellen, oder aber
- R⁶ und R⁷: gemeinsam für eine α- oder β-ständige Methylengruppe und R⁶' für ein Wasserstoffatom oder
- R⁶ und R⁶': gemeinsam für eine Ethylen- oder Methylengruppe und R⁷ für ein Wasserstoffatom.
- R⁹ und R¹⁰: jeweils für ein Wasserstoffatom oder eine gemeinsame Bindung,
- R¹¹ und R¹²: jeweils für ein Wasserstoffatom oder eine gemeinsame Bindung,
- R¹³: für eine Methyl- oder Ethylgruppe,
- R¹⁵: für ein Wasserstoffatom oder einen C₁-C₃-Alkylrest,
- R¹⁶ und R¹⁶': unabhängig voneinander für ein Wasserstoffatom, einen C₁-C₃-Alkylrest oder einen C₂-C₄-Alkenylrest oder gemeinsam für eine C₁-C₃-Alkylidengruppe
- R¹⁵ und R¹⁶: für eine gemeinsame Bindung sowie R¹⁶' für ein Wasserstoffatom oder einen C₁-C₃-Alkylrest oder
- R¹⁵ und R¹⁶: gemeinsam für einen Ring der Teilformel 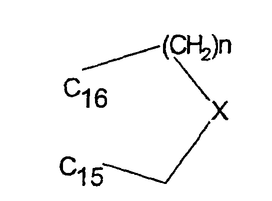
worin n = 1 und 2 und X eine Methylengruppe oder ein Sauerstoffatom bedeuten sowie
- R¹⁶': für ein Wasserstoffatom,
- R^{17¹}: für ein Wasserstoffatom oder einen C₁-C₃-Alkylrest,
- R^{17²}: für ein Wasserstoffatom, einen C₁-C₃-Alkylrest oder einen C₂-C₄-Alkenylrest
- R^{17^{1'}} und R^{17^{2'}}: jeweils für ein Wasserstoffatom oder für eine gemeinsame Bindung,
- R²¹: für ein Wasserstoffatom oder einen C₁-C₃-Alkylrest,
- R²¹': für eine Hydroxygruppe stehen.

Die Schlangenlinien 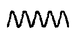 in den allgemeinen Formeln der vorliegenden Erfindung bedeuten, daß der betreffende Substituent sich in der α- oder β-Position an dem entsprechenden Kohlenstoffatom befinden kann.

Bei den vorstehend als mögliche Substituenten genannten C₁-C₃-Alkylgruppen kann es sich um eine Methyl-, Ethyl-, n-Propyl- oder i-Propyl- und bei den C₁-C₄-Alkylgruppen zusätzlich um eine n-Butyl-, i-Butyl- oder tert.-Butylgruppe handeln. Eine Methyl- oder Ethylgruppe ist in allen Fällen bevorzugt.
Im Falle des C₂-C₄-Alkenylrestes für R¹⁶, R¹⁶' und/oder R^{17²} handelt es sich um einen Vinyl-, Allyl- oder But-3-enylrest; der Vinylrest ist bevorzugt.

### Spezielle Gestagene:

Bevorzugt gemäß vorliegender Erfindung sind Kombinationen aus mindestens einem Gestagen und einem β-Cyclodextrin oder γ-Cyclodextrin, oder Derivaten dieser Cyclodextrine, die durch Veretherung oder Veresterung freier alkoholischer Funktionen der Cyclodextrine erhalten werden,
wobei die Gestagene zur Gruppe der Formel I zählen:
in der
- R³: für ein Sauerstoffatom oder zwei Wasserstoffatome, und/oder
- R⁶: für ein Wasserstoffatom oder für einen α- oder β-ständigen C₁-C₄-Alkyfrest,
wenn R⁶' und R⁷ Wasserstoffatome darstellen, oder aber
- R⁶': für ein Wasserstoff-, Chlor- oder Bromatom oder für einen C₁-C₄-Alkylrest,
wenn R⁶' und R⁷ eine gemeinsame zusätzliche Bindung darstellen
und/oder
- R¹⁶ und R¹⁶': jeweils für ein Wasserstoffatom, jeweils für eine Methylgruppe oder der eine dieser beiden Substituenten für eine C₁-C₄-Alkyl- oder eine Vinylgruppe und der andere dieser beiden Substituenten für ein Wasserstoffatom stehen,
oder
beide gemeinsam eine C1-C3-Alkyliden gruppe bilden
und/oder
- R^{17¹} und R^{17²}: unabhängig voneinander für ein Wasserstoffatom oder eine Methylgruppe
und/oder
- R^{17^{1'}} und R^{17²}: jeweils für ein Wasserstoffatom oder eine gemeinsame Bindung
und/oder
- R²¹: für ein Wasserstoffatom oder einen C₁-C₃-Alkylrest sowie R²¹' für ein Wasserstoffatom oder eine Hydroxygruppe
stehen,
sowie die anderen Substituenten alle die in Formel (I) angegebenen Bedeutungen haben können.

Mehr bevorzugt sind Kombinationen aus mindestens einem Gestagen und einem β-Cyclodextrin oder γ-Cyclodextrin, oder Derivaten dieser Cyclodextrine, die durch Veretherung oder Veresterung freier alkoholischer Funktionen der Cyclodextrine erhalten werden,
wobei das Gestagen zur Gruppe der folgenden Substanzen zählt:
14,17-Ethano-19-norpregna-4,9-dien-3,20-dion;
14,17-Ethano-19-norpregna-4,6-dien-3,20-dion;
14,17-Ethano-19-norpregna-4,15-dien-3,20-dion
14,17-Ethano-19-norpregna-4,6,15-trien-3,20-dion
14,17-Ethano-19-norpregna-4,9,15-trien-3,20-dion
21-Methyl-14,17-ethano-19-norpregn-4-en-3,20-dion;
21-Methyl-14,17-ethano-19-norpregna-4,9-dien-3,20-dion;
21-Methyl-14,17-ethano-19-norpregna-4,6-dien-3,20-dion;
21-Methyl-14,17-ethano-19-norpregna-4,15-dien-3,20-dion
21-Methyl-14,17-ethano-19-norpregna-4,9,15-trien-3,20-dion
14,17-Etheno-19-norpregn-4-en-3,20-dion;
14,17-Etheno-19-norpregna-4,6-dien-3,20-dion;
14,17-Etheno-19-norpregna-4,9-dien-3,20-dion;
21-Methyl-14,17-etheno-19-norpregn-4-en-3,20-dion
21-Methyl-14,17-etheno-19-norpregna-4,6-dien-3,20-dion
21-Methyl-14,17-etheno-19-norpregna-4,9-dien-3,20-dion;
21-Methyl-14,17-etheno-19-norpregna-4,9,11-trien-3,20-dion
21-Hydroxy-14,17-etheno-19-norpregn-4-en-3,20-dion
21-Hydroxy-14,17-etheno-19-norpregna-4,9-dien-3,20-dion
17¹-Methyl-14,17-etheno-19-norpregn-4-en-3,20-dion
17¹-Methyl-14,17-etheno-19-norpregna-4,6-dien-3,20-dion
17²-Methyl-14,17-etheno-19-norpregn-4-en-3,20-dion
17²-Methyl-14,17-etheno-19-norpregna-4,9-dien-3,20-dion
15β,16α-Dimethyl-14,17-etheno-19-norpregn-4-en-3,20-dion
6-Methyl-14,17-ethano-19-norpregna-4,6-dien-3,20-dion;
6-Chlor -14,17-ethano-19-norpregna-4,6-dien-3,20-dion;
6α-Methyl-14,17-ethano-19-norpregn-4-en-3,20-dion;
6,21-Dimethyl-14,17-ethano-19-norpregna-4,6-dien-3,20-dion;
15β,16α-Dimethyl-14,17-ethano-19-norpregn-4-en-3,20-dion
6-Chlor-21-methyl-14,17-ethano-19-norpregna-4,6-dien-3,20-dion;
16α-Methyl-14,17-ethano-19-norpregn-4-en-3,20-dion;
16α-Methyl-14,17-ethano-19-norpregna-4,6-dien-3,20-dion;
16α-Methyl-14,17-ethano-19-norpregna-4,9-dien-3,20-dion;
16α,21-Dimethyl-14,17-ethano-19-norpregna-4,9-dien-3,20-dion
21-Hydroxy-16α-methyl-14,17-ethano-19-norpregn-4-en-3,20-dion
16α-Ethyl-14,17-ethano-19-norpregn-4-en-3,20-dion;
16α-Ethenyl-14,17-ethano-19-norpregn-4-en-3,20-dion;
16-Methyl-14,17-ethano-19-norpregna-4,15-dien-3,20-dion
(17¹*R*)-17¹-Methyl-14,17-ethano-19-norpregn-4-en-3,20-dion
(17¹*S*)-17¹-Methyl-14,17-ethano-19-norpregn-4-en-3,20-dion
(17¹*R*)-17¹-Methyl-14,17-ethano-19-norpregna-4,9-dien-3,20-dion
(17¹*S*)-17¹-Methyl-14,17-ethano-19-norpregna-4,9-dien-3,20-dion
(17²*R*)-17²-Methyl-14,17-ethano-19-norpregn-4-en-3,20-dion
(17²*R*)-17²-Methyl-14,17-ethano-19-norpregna-4,6-dien-3,20-dion
(17²*R*)-17²-Methyl-14,17-ethano-19-norpregna-4,9-dien-3,20-dion
(17²*R*)-17²,21-Dimethyl-14,17-ethano-19-norpregna-4,6-dien-3,20-dion
(17²*R*)-17²,21-Dimethyl-14,17-ethano-19-norpregna-4,9-dien-3,20-dion
(17²*R*)-17²,21-Dimethyl-14,17-ethano-19-norpregna-4,9,11-trien-3,20-dion
16-Methylen-14,17-ethano-19-norpregn-4-en-3,20-dion
16-Methylen-14,17-ethano-19-norpregna-4,6-dien-3,20-dion
16-Methylen-14,17-ethano-19-norpregna-4,9-dien-3,20-dion
21-Hydroxy-14,17-ethano-19-norpregn-4-en-3,20-dion;
21-Hydroxy-14,17-ethano-19-norpregna-4,6-dien-3,20-dion;
21-Hydroxy-14,17-ethano-19-norpregna-4,9-dien-3,20-dion;
21-Hydroxy-14,17-ethano-19-norpregna-4,9,15-trien-3,20-dion
(21*R*)-21-Hydroxy-21-methyl-14,17-ethano-19-norpregn-4-en-3,20-dion;
(21 *S*)-21-Hydroxy-21-methyl-14,17-ethano-19-norpregn-4-en-3,20-dion;
(21*R*)-21-Hydroxy-21-methy)-14,17-ethano-19-norpregna-4,9-dien-3,20-dion;
(21*S*)-21-Hydroxy-21-methyl-14,17-ethano-19-norpregna-4,9-dien-3,20-dion;
(21*R*)-21-Hydroxy-21-methyl-14,17-ethano-19-norpregna-4,6-dien- 3,20-dion;
(21*S*)-21-Hydroxy-21-methyl-14,17-ethano-19-norpregna-4,6-dien- 3,20-dion;
(21*R*)-21-Hydroxy-21-methyl-14,17-ethano-19-norpregna-4,9,15-trien-3,20-dion
(21*S*)-21-Hydroxy-21-methyl-14,17-ethano-19-norpregna-4,9,15-trien-3,20-dion
14,17-Ethano-18a-homo-19-norpregn-4-en-3,20-dion
14,17-Ethano-18a-homo-19-norpregna-4,6-dien-3,20-dion
14,17-Ethano-18a-homo-19-norpregna-4,9-dien-3,20-dion
14,17-Ethano-18a-homo-19-norpregna-4,15-dien-3,20-dion
21-Methyl-14,17-ethano-18a-homo-19-norpregn-4-en-3,20-dion
21-Methyl-14,17-ethano-18a-homo-19-norpregna-4,6-dien-3,20-dion
21-Methyl-14,17-ethano-18a-homo-19-norpregna-4,9-dien-3,20-dion
(21*R*)-21-Hydroxy-21-methyl-14,17-ethano-18a-homo-19-norpregn-4-en-3,20-dion
(21*S*)-21-Hydroxy-21-methyl-14,17-ethano-18a-homo-19-norpregn-4-en-3,20-dion
(21*R*)-21-Hydroxy-21-methyl-14,17-ethano-18a-homo-19-norpregna-4,9-en-3,20-dion
(21*S*)-21-Hydroxy-21-methyl-14,17-ethano-18a-homo-19-norpregna-4,9-en-3,20-dion
(21*R*)-21-Hydroxy-21-methyl-14,17-ethano-18a-homo-19-norpregna-4,6-en-3,20-dion
(21*S*)-21-Hydroxy-21-methyl-14,17-ethano-18a-homo-19-norpregna-4,6-en-3,20-dion

### Wirksamkeit der Kombination:

Nach oraler Gabe bildet sich am gastrointestinalen Resorptions - Ort aus dem Komplex aus Gestagen und Zuckerderivat ein Gleichgewicht zwischen dem nicht - dissoziierten Komplex und den Einzelkomponenten. Dabei wird durch Verdrängung des Gestagens aus dem komplexierenden Zuckerderivat der freie Wirkstoff schnell freigesetzt und gelangt zur Resorption. Das Zuckerderivat hingegen wird nicht resorbiert und unverändert über den Darm ausgeschieden. Die pharmakologische Wirkung des Gestagens ist in WO96/20209 beschrieben.
Im Gestagenrezeptor - Bindungstest auf gestagene Wirkung unter Verwendung von Cytosol aus Kaninchenuterushomogenat und von ³H-Progesteron als Bezugssubstanz zeigt das Gestagen eine sehr starke Affinität zum Gestagenrezeptor. Im Schwangerschaftserhaltungstest an der Ratte zeigen die Gestagene der allgemeinen Formel (I) eine sehr hohe gestagene Wirksamkeit.

Zusätzlich zur sehr hohen gestagene Wirksamkeit im Schwangerschaftserhaltungstest zeigen die Gestagene der allgemeinen Formel I im Gegensatz zur bereits bekannten Verbindung 14,17-Ethano-19-norpregn-4-en-3,20-dion aber größtenteils auch nach oraler Gabe eine gute gestagene Wirkung.
Aufgrund ihrer hohen gestagenen Wirksamkeit können die Gestagene der allgemeinen Formel (I) beispielsweise allein oder in Kombination mit Estrogenen in Präparaten zur Kontrazeption verwendet werden. Aber auch alle anderen heutzutage für Gestagene bekannten Verwendungsmöglichkeiten stehen dem neuen Komplex offen.

Die Dosierung der erfindungsgemäßen Komplexen in Kontrazeptionspräparaten soll vorzugsweise 0,01 bis 2 mg, berechnet als freies Gestagen pro Tag betragen. Geeignete Dosierungen können routinemäßig bestimmt werden, zum Beispiel durch Bestimmung der Bioäquivalenz gegenüber einem bekannten Gestagen für eine bestimmte Verwendung, beispielsweise eine Menge, die bioäquivalent zu 0,030 bis 0,150 mg Levonorgestrel für die Kontrazeption ist. Diese Eichung gilt auch für die weiteren Angaben der Dosierungen zu den Gestagenen.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt. Neben der oralen ist z.B. auch eine transdermale Verabreichung möglich.

Als Estrogene kommen vorzugsweise synthetische Estrogene wie Ethinylestradiol, 14α,17α-Ethano-1,3,5(10)-estratrien-3,17β-diol (WO 88/01275) oder 14α,17α-Ethano-1,3,5(10)-estratrien-3,16α,17β-triol (WO 91/08219) in Betracht.
Das Estrogen wird in einer Menge verabfolgt, die der von 0,01 bis 0,05 mg Ethinylestradiol entspricht.

Die neuen Kombinationen aus mindestens einem Gestagen der Formel I und aus einem β-Cyclodextrin oder γ-Cyclodextrin, oder Derivaten dieser Cyclodextrine, können auch in Präparaten zur Behandlung gynäkologischer Störungen und zur Substitutionstherapie eingesetzt werden. Wegen ihres günstigen Wirkungsprofils sind die erfindungsgemäßen Kombinationen besonders gut geeignet zur Behandlung prämenstrueller Beschwerden, wie Kopfschmerzen, depressive Verstimmung, Wasserretention und Mastodynie. Die Tagesdosis bei der Behandlung prämenstrueller Beschwerden liegt bei etwa 1 bis 20 mg, berechnet als freies Gestagen.

Die Formulierung der pharmazeutischen Präparate auf Basis der neuen Kombinationen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.
Für die bevorzugte orale Applikation kommen insbesondere Tabletten, Dragees, Kapseln, Pillen, Suspensionen oder Lösungen in Frage.
Für die transdermaleApplikation sind insbesondere Matrix- oder Membranpflaster geeignet.
Die Kombinationen mit Verbindungen der allgemeinen Formel (I) können auch kontinuierlich durch ein intrauterines Freisetzungssystem (IUD) appliziert werden; die Freisetzungsrate der aktiven Verbindung(en) wird dabei so gewählt, daß die täglich freigesetzte Dosis innerhalb der bereits angegebenen Dosierungsbereiche liegt.
Die Herstellung der Gestagene ist in der WO 96/20209 (Publikationsdatum 4. Juli 1996) ausführlich beschrieben.
Bevorzugt ist eine Kombination mit dem Gestagen (21S)-21-Hydroxy-21-methyl-14,17-ethano-19-norpregna-4,9,15-triene-3,20-dion.

### Cyclodextrine:

β-Cyclodextrin, γ-Cyclodextrin und Derivate dieser Cyclodextrine, die durch Veretherung oder Veresterung freier alkoholischer Funktionen der Cyclodextrine erhalten werden, sind beschrieben in J. Pharm. Sci. 74 (1985) S. 987-990 oder Int. J. Pharm. 29 (1986) S. 73-82.

Mehr bevorzugt ist eine Kombination aus einem Gestagen und einem Cyclodextrin, wobei das Cyclodextrin ein β-Cyclodextrin ist.

Am meisten bevorzugt ist die Kombination aus dem Gestagen (21S)-21-Hydroxy-21-methyl-14,17-ethano-19-norpregna-4,9,15-triene-3,20-dion und dem β-Cyclodextrin.

### Vorteile:

Werden Gestagene, insbesondere (21S)-21-Hydroxy-21-methyl-14,17-ethano-19-norpregna-4,9,15-triene-3,20-dion mit Hilfsstoffen wie Lactose, Maisstärke, Mannitol, mikrokistalline Cellulose, Polyvidon, Hydroxypropylmethylcellulose, Dicalciumphosphat und Maltodextrin vermengt, so ist ein beschleunigter Abbau festzustellen. Hierbei handelt es sich um eine Acyloinumlagerung. Dabei entsteht eine Mischung aus zwei Paaren von Diastereomeren mit jeweils getauschten Positionen der Ketogruppe und der Hydroxylgruppe am C₂₀ und C₂₁ Atom. Von den vier möglichen Strukturen entspricht lediglich eine der zuvor genannten Substanz (21S)-21-Hydroxy-21-methyl-14,17-ethano-19-norpregna-4,9,15-triene-3,20-dion.
So verringert sich bei einer Lagerung bei 25 °C (60% rel. Feuchte) über 3 Monate der Gehalt an nicht komplexiertem (21S)-21-Hydroxy-21-methyl-14,17-ethano-19-norpregna-4,9,15-triene-3,20-dion auf unter 90% des Startwertes, wenn die Substanz entweder (i) mit den Hilfsstoffen Lactose, Maisstärke, modifizierte Maisstärke, Polyvidon 25,000 und Magnesiumstearat oder (ii) mit den Hilfsstoffen Mannitol, Hydroxypropylmethylcellulose und Magnesiumstearat tablettiert wird. Auch die Formulierungen mit den Hilfsstoffen (iii) direkttablettierbares Mannitol oder (iv) mikrokristalline Cellulose und Magnesiumstearat oder (v) Glyceryltribehenat zeigen einen vergleichbaren Abbau der Substanz (21S)-21-Hydroxy-21-methyl-14,17-ethano-19-norpregna-4,9,15-triene-3,20-dion.
Durch die erfindungsgemäße Kombination (Komplexierung des Gestagens mit β-Cyclodextrin) lassen sich Tabletten erhalten, die trotz Lagerung bei den kritischen Temperaturen einen Wirkstoffgehalt aufweisen, welcher nach 6-monatiger Lagerung bei 40°C, 75% relativer Feuchte im offenen Standglas noch über 90% des Startwertes bleibt.

### Weitere Ausführungsformen zu Cyclodextrinen

Vorteilhaft ist eine erfindungsgemäße Kombination, bei der das Cyclodextrin und das Gestagen
beim β-Cyclodextrin in einem Komplex 1 : n (Gestagen : Cyclodextrin, n ≥ 1) vorliegt , bevorzugt ist ein Verhältnis von 1 : 2 (Gestagen : Cyclodextrin), und
beim γ-Cyclodextrin ebenfalls in einem Komplex 1 : n (n ≥ 1) (Gestagen : Cyclodextrin) vorliegt, bevorzugt ist ein Verhältnis von 1 : 2 (Gestagen : Cyclodextrin).

Neben der Stabilitätserhöhung läßt sich die Stöchiometrie der Komplexbildung ermitteln. Dabei ist offensichtlich, daß die Komplexbildung bei den Komplexen (21S)-21-Hydroxy-21-methyl-14,17-ethano-19-norpregna-4,9,15-triene-3,20-dion und γ-Cyclodextrin im Verhältnis 1 :1 bis 1 : 2 (Gestagen : Cyclodextrin) stattfindet. Vorteilhaft ist ein Komplexierungsverhältnis von 1 : 2 (Gestagen : Cyclodextrin).
Bei dem Komplex aus (21S)-21-Hydroxy-21-methyl-14,17-ethano-19-norpregna-4,9,15-triene-3,20-dion und β-Cyclodextrin liegt ein Verhältnis von 1 : 2 (Gestagen : Cyclodextrin) vor.
Derartige Komplexe weisen ein zum Teil niedrigeres Löslichkeitsprodukt als das Steroid allein auf. Somit können die Komplexe durch eine Fällungsreaktion (z.B. Copräzipitation) dargestellt werden.

### Weitere Ausführungsformen als Arzneimittel

Bevorzugt ist die erfindungsgemäße Kombination als Arzneimittel. Die Wirkung der Substanzen ist zuvor beschrieben (vgl. WO96/20209).

Mehr bevorzugt ist ein Arzneimittel oder ein pharmazeutisches Präparat, enthaltend einen erfindungsgemäße Kombination einschließlich pharmazeutischer Trägerstoffe und Hilfsstoffe.

Noch mehr bevorzugt ist ein Arzneimittel oder pharmazeutisches Präparat enthaltend eine erfindungsgemäße Kombination zur peroralen, oralen, parenteralen, transdermalen, pulmonalen, nasalen, rektalen, vaginalen oder intrauterinen Anwendung.

Die Erfindung betrifft weiterhin die Verwendung von erfindungsgemäßen Kombinationen zusammen mit pharmakologischen Hilfs- und Trägerstoffen, die physiologisch verträglich sind, zur Herstellung eines Medikaments zur Behandlung von klimakterischen Beschwerden. Solche Hilfs- und Trägerstoffe sind in Remington's Pharmaceutical Science, 15^{th} ed. Mack Publishing Company, Easton Pennsylvania (1980) beschrieben.

Die erfindungsgemäßen Kombinationen zeigen die Wirkung in den zuvor genannten Test bei Konzentrationen von 0,1 bis 1000 ng/ml des Gestagens.

Für die therapeutische Wirkung ist die geeignete Dosis unterschiedlich und hängt beispielsweise von der verwendeten Kombinationen mit Gestagenen der allgemeinen Formel I, dem Wirt, der Art der Verabreichung und der Art und der Schwere der zu behandelnden Zustände ab. Im allgemeinen sind jedoch bei Tieren zufriedenstellende Resultate zu erwarten bei täglichen Dosen an Gestagen von 1 bis 3000 µg/kg Tierkörpergewicht. Bei größeren Säugetieren, beispielsweise Menschen, beträgt eine empfohlene tägliche Dosis an Gestagen 0,1 bis 200 mg. Bevorzugt sind Werte von 0,3 bis 60mg pro Tag, mehr bevorzugt 1 bis 20 mg pro Tag und am meisten bevorzugt 2 bis 10 mg pro Tag.

Die Erfindung liefert weiterhin
(i) die Verwendung einer der erfindungsgemäßen Kombinationen zur Herstellung eines Medikaments zur Behandlung von klimakterischen Beschwerden;
(ii) ein Verfahren zur Behandlung von klimakterische Beschwerden, welches Verfahren eine Verabreichung einer Kombinationsmenge gemäß der Erfindung umfaßt, wobei die Menge die Krankheit unterdrückt, und wobei die Kombinationsmenge einem Patienten gegeben wird, der ein solches Medikament benötigt;
(iii) eine pharmazeutische Zusammensetzung zur Behandlung von klimakterische Beschwerden, welche Behandlung eine der erfindungsgemäßen Kombinationen und wenigstens einen pharmazeutischen Hilfs- und/oder Trägerstoff umfaßt.

Die Erfindung liefert weiterhin
(i) die Verwendung einer der erfindungsgemäßen Kombinationen zur Herstellung eines Medikaments zur Behandlung prämenstrueller Beschwerden, wie Kopfschmerzen, depressive Verstimmung, Wasserretention und Mastodynie;
(ii) ein Verfahren zur Behandlung prämenstrueller Beschwerden, wie Kopfschmerzen, depressive Verstimmung, Wasserretention und Mastodynie, welches Verfahren eine Verabreichung einer Kombinationsmenge gemäß der Erfindung umfaßt, wobei die Menge die Krankheit unterdrückt, und wobei die Kombinationsmenge einem Patienten gegeben wird, der ein solches Medikament benötigt;
(iii) eine pharmazeutische Zusammensetzung zur Behandlung prämenstrueller Beschwerden, wie Kopfschmerzen, depressive Verstimmung, Wasserretention und Mastodynie, welche Behandlung eine der erfindungsgemäßen Kombinationen und wenigstens einen pharmazeutischen Hilfs- und/oder Trägerstoff umfaßt.

Die Tagesdosis bei der Behandlung prämenstrueller Beschwerden liegt bei etwa 1 bis 20 mg, berechnet als freies Gestagen.

### Weitere Ausführungsformen als orales Kontrazeptivum

Die Erfindung umfaßt eine erfindungsgemäße Kombination für die Fertilitätskontrolle.

Aufgrund ihrer hohen gestagenen Wirksamkeit können die neuen Kombinationen mit Gestagenen der allgemeinen Formel (I) beispielsweise allein oder in Kombination mit Estrogenen in Präparaten zur Kontrazeption verwendet werden. Aber auch alle anderen heutzutage für Gestagene bekannten Verwendungsmöglichkeiten stehen den neuen Verbindungen offen.

Die Dosierung der erfindungsgemäßen Kombination in Kontrazeptionspräparaten soll vorzugsweise 0,01 bis 2 mg pro Tag, berechnet als freies Gestagen betragen.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen vorzugsweise synthetische Estrogene wie Ethinylestradiol, 14α,17α-Ethano-1,3,5(10)-estratrien-3,17β-diol (WO 88/01275) oder 14α,17α-Ethano-1,3,5(10)-estratrien-3,16α,17β-triol (WO 91/08219) in Betracht.

Das Estrogen wird in einer Menge verabfolgt, die der von 0,01 bis 0,05 mg Ethinylestradiol entspricht.

Schließlich können die neuen Kombinationen auch als gestagene Komponente in den neuerdings bekannt gewordenen Zusammensetzungen für die weibliche Fertilitätskontrolle, die sich durch die zusätzliche Verwendung eines kompetitiven Progesteronantagonisten auszeichnen, zum Einsatz kommen (H.B. Croxatto und A.M. Salvatierra in Female Contraception and Male Fertility Regulation, ed. by Runnebaum, Rabe & Kiesel - Vol. 2, Advances in Gynecological and Obstetric Research Series, Parthenon Publishing Group - 1991, Seite 245).
Die Dosierung liegt im bereits angegebenen Bereich, die Formulierung kann wie bei konventionellen OC-Präparaten erfolgen. Die Applikation des zusätzlichen, kompetitiven Progesteronantagonisten kann dabei auch sequentiell vorgenommen werden.

### Weitere Ausführungsformen als Stabilisierungsverfahren

Vorteilhaft ist ein Verfahren zur Stabilisierung eines Gestagens nach der Formel I unter Verwendung von einem β-Cyclodextrin oder γ- Cyclodextrin oder Derivaten dieser Cyclodextrine, die durch Veretherung oder Veresterung freier alkoholischer Funktionen der Cyclodextrine erhalten werden. Der bevorzugte Komplex aus Gestagen und Cyclodextrin ist der Komplex aus (21S)-21-Hydroxy-21-methyl-14,17-ethano-19-norpregna-4,9,15-triene-3,20-dion und β-Cyclodextrin.

Bevorzugt ist ein Verfahren zum Komplexieren von einem Gestagen und von einem β-Cyclodextrin oder γ-Cyclodextrin unter Verreiben als Trockenmischung. Röntgenspektren des Pulvers, welches als Trockenmischung hergestellt wurde, zeigen, daß die Komplexierung partiell bereits vorliegt, jedoch noch nicht vollständig vollzogen worden ist. Diese Komplexierung bereits als Trockenmischung ist überraschend.

Mehr bevorzugt ist die Darstellung der Komplexe durch Fällungsreaktion, z.B. Copräzipitation, indem zu einer wässrigen Cyclodextrinlösung eine ethanolische Lösung des Gestagens zugetropft wird. Die durch Fällung hergestellten Komplexe aus Gestagenen und Cyclodextrin können vor der Formulierung zum Arzneimittel durch geeignete Vermahlungstechniken, z.B. die der Luftstrahlvermahlung, auf die gewünschte Partikelgrößenverteilung gebracht werden.

Bevorzugt zur Herstellung der Formulierung ist eine Verkapselung oder Granulierung und anschließende Tablettierung.
Mehr bevorzugt ist ein Verfahren der Direkttablettierung des Komplexes aus einem Gestagen mit β-Cyclodextrin oder γ-Cyclodextrin unter Zugabe von pharmazeutisch verträglichen Hilfsstoffen. Dabei wird auf Granulierungsschritte verzichtet. Ein Granulierungsschritt beinhaltet die Gefahr, daß der Cyclodextrin - Komplex zerstört würde, indem das Steroid aus dem Cyclodextrin - Wirt durch Hilfsstoffmoleküle als Gast verdrängt würde.
Es wurde daher eine Direkttablettierung unter Zugabe der Hilfsstoffe mikrokristalline Cellulose, Lactose, Croscarmellose-Na, hochdisperses Siliciumdioxid und Magnesiumstearat durchgeführt.

### Beispiel

Die Komplexe aus (21S)-21-Hydroxy-21-methyl-14,17-ethano-19-norpregna-4,9,15-triene-3,20-dion und aus β- und γ-Cyclodextrin wurden in folgender Weise hergestellt: 19 mmol des Cyclodextrins wurden in 610 ml 45°C warmem Wasser gelöst und innerhalb von 30 min 7,6 mmol ZK 187226, gelöst in 10 ml Ethanol zugetropft. Mit weiteren 5 ml Ethanol wurde nachgespült, auf Raumtemperatur abkühlen lassen, 24h bei RT gerührt, 2h im Eisbad (2 °C) gerührt und der Niederschlag über eine G2-Fritte abgesaugt. Der erhaltene Komplex wurde anschließend noch 2 mal mit je 50 ml Eiswasser und einmal mit eiskaltem Aceton gewaschen. Nach Trocknung im Exsikator über Phosphorpentoxid wurde der Komplex durch Karl-Fischer-Wasserbestimmung, HPLC, DSC und Röntgenpulverdiffraktometrie charakterisiert.

Die Tatsache, daß selbst nach der vergleichend durchgeführten bloßen Verreibung eine deutliche Veränderung sowohl im Rötgenpulverspektrum als auch in der DSC beobachtet wird, deutet darauf hin, daß eine partielle, aber nicht vollständige Komplexierung bereits bei der Verreibung von (21S)-21-Hydroxy-21-methyl-14,17-ethano-19-norpregna-4,9,15-triene-3,20-dion mit β - oder γ-Cyclodextrin stattfindet.
Aus den hergestellten Cyclodextrinkomplexen von (21S)-21-Hydroxy-21-methyl-14,17-ethano-19-norpregna-4,9,15-triene-3,20-dion wurden nun nach Vermahlung der Komplexe Tabletten hergestellt. Für die Tablettierung ist von Bedeutung, daß sie als Direkttablettierung durchgeführt wird, ohne einen Granulierschritt. Ein solcher Granulierprozess würde nämlich die Gefahr beinhalten, daß die Cyclodextrinkomplexe zerstört werden, indem (21S)-21-Hydroxy-21-methyl-14,17-ethano-19-norpregna-4,9,15-triene-3,20-dion aus dem Cyclodextrin Wirt durch Hilfsstoffmoleküle als Gast verdrängt würde.
Es wurde daher eine Direkttablettierung unter Zugabe der Hilfsstoffe mikrokristalline Cellulose, Lactose, Croscarmellose-Na, hochdisperses Siliciumdioxid und Magnesiumstearat durchgeführt.
Die hergestellten Tabletten sowie eine aus nicht komplexiertem Wirkstoff hergestellte Formulierung und die Komplexe aus (21S)-21-Hydroxy-21-methyl-14,17-ethano-19-norpregna-4,9,15-triene-3,20-dion und β- und γ-Cyclodextrin wurden zur Stabilitätsprüfung eingelagert und der Gehalt an (21S)-21-Hydroxy-21-methyl-14,17-ethano-19-norpregna-4,9,15-triene-3,20-dion (bei den Tabletten bezogen auf den Sollgehalt von 0,1mg Wirkstoff pro Tablette, bei den Komplexen als Massenprozent) nach 1,5 und 3-monatiger Lagerung bestimmt. Die Ergebnisse sind in den Tabellen 1 bis 5 dargestellt.

Die aus den β-Cyclodextrin- und γ-Cyclodextrin-Clathraten zeigen im Vergleich zu den mit unkomplexiertem Wirkstoff hergestellten Tabletten eine deutlich verbesserte Stabilität. Das β-Cyclodextrin-Clathrat zeigt die beste Stabilisierung, ist in guter Qualität herstellbar und auch ökonomisch gegenüber γ-Cyclodextrin zu bevorzugen. Aufgrund der Ergebnisse der 3 Monats-Auslagerung ergibt sich für die mit dem Komplex aus (21S)-21-Hydroxy-21-methyl-14,17-ethano-19-norpregna-4,9,15-triene-3,20-dion und β-Cyclodextrin hergestellten Tabletten eine für eine Marktformulierung ausreichende Stabilität.

**Tabelle 1:**

| β-Cyclodextrin-Clathrat Tabletten | | | | | | | |
|---|---|---|---|---|---|---|---|
| Monate | -18°C | +25°C | +25°C, 60% r.F. | +40°C | +40°C, 75% r.F. | +60°C | +60°C, 75% r.F. |
| 0 | 97.0% (1.0%) | - | - | - | - | - | - |
| 1.5 | 97.4% (0.8%) | 97.0% (0.6%) | 96.7% (0.7%) | 95.5% (1.8%) | 93.5% (0.9%) | 92.9% (0.7%) | 89.7% (0.8%) |
| 3 | 97.0% (0.8%) | 97.1% (1.0%) | 96.6% (0.7%) | 95.2% (0.5%) | 91.8% (1.0%) | 92.6% (1.2%) | 89.0% (1.7%) |

**Tabelle 2:**

| γ-CD-Clathrat Tabletten | | | | | | | |
|---|---|---|---|---|---|---|---|
| Monate | -18°C | +25°C | +25°C, 60% r.F. | +40°C | +40°C, 75% r.F. | +60°C | +60°C, 75% r.F. |
| 0 | 93.0% (8.3%) | - | - | - | - | - | - |
| 1.5 | 97.9% (2.6%) | 99.4% (2.5%) | 98.8% (2.7%) | 98.5% (3.6%) | 95.1% (7.3%) | 90.0% (4.7%) | 85.1% (5.1%) |
| 3 | 99.8% (4.2%) | 96.5% (3.9%) | 99.9% (5.7%) | 97.9% (3.4%) | 88.7% (3.0%) | 77.1% (4.7%) | 79.1% (1.6%) |

**Tabelle 3:**

| (21S)-21-Hydroxy-21-methyl-14,17-ethano-19-norpregna-4,9,15-triene-3,20-dion Tabletten | | | | | | | |
|---|---|---|---|---|---|---|---|
| Monate | -18°C | +25°C | +25°C. 60% r.F. | +40°C | +40°C, 75% r.F. | +60°C | +60°C, 75% r.F. |
| 0 | 100.1% (0.8%) | - | - | - | - | - | - |
| 1.5 | nicht untersucht | 90.6% (0.5%) | 80.3% (0.3%) | 26.6% (0.8%) | 27.9% (1.1%) | 0.3% (1.7%) | 0.1% (14.3%) |
| 3 | 101.2% (0.5%) | 80.1% (0.6%) | 63.9% (1.0%) | 8.5% (5.2%) | 14.1% (4.1%) | 0.3% (68.0%) | 0.1% (65.6%) |

**Tabelle 4:**

| (21S)-21-Hydroxy-21-methyl-14,17-ethano-19-norpregna-4,9,15-triene-3,20-dion - β-Cyclodextrin-Komplex Gehalt in % | | | | | | | |
|---|---|---|---|---|---|---|---|
| Monate | -18°C | +25°C | +25°C, 60% r.F. | +40°C | +40°C, 75% r.F. | +60°C | +60°C, 75% r.F. |
| 0 | 12.5% (0.3%) | - | - | - | - | - | - |
| 1.5 | 12.5% (0.7%) | nicht untersucht | 12.5% (0.4%) | nicht untersucht | 12.4% (0.9%) | nicht untersucht | 12.3% (0.8%) |
| 3 | 12.5% (0.5%) | nicht untersucht | 12.5% (0.3%) | nicht untersucht | 12.6% (0.3%) | nicht untersucht | 12.3% (0.5%) |

**Tabelle 5:**

| (21S)-21-Hydroxy-21-methyl-14,17-ethano-19-norpregna-4,9,15-triene-3,20-dion - γ-Cyclodextrin-Komplex Gehalt in % | | | | | | | |
|---|---|---|---|---|---|---|---|
| Monate | -18°C | +25°C | +25°C, 60% r.F. | +40°C | +40°C, 75% r.F. | +60°C | +60°C, 75% r.F. |
| 0 | 13.6% (2.8%) | - | - | - | - | - | - |
| 1.5 | 13.8% (1.2%) | nicht untersucht | 13.7% (0.8%) | nicht untersucht | 13.3% (0.5%) | nicht untersucht | 12.0% (1.3%) |
| 3 | 13.9% (2.5%) | nicht untersucht | 13.5% (1.3%) | nicht untersucht | 13.0% (0.5%) | nicht untersucht | 10.0% (1.1%) |
| r.F. = relative Luftfeuchte, eingestellt in der Klimakammer | | | | | | | |

## Patentansprüche

1. Kombination aus mindestens einem Gestagen und einem β-Cyclodextrin oder γ-Cyclodextrin oder Derivaten dieser Cyclodextrine, die durch Veretherung oder Veresterung freier alkoholischer Funktionen der Cyclodextrine erhalten werden.
wobei die Gestagene zur Gruppe der Formel I zählen: 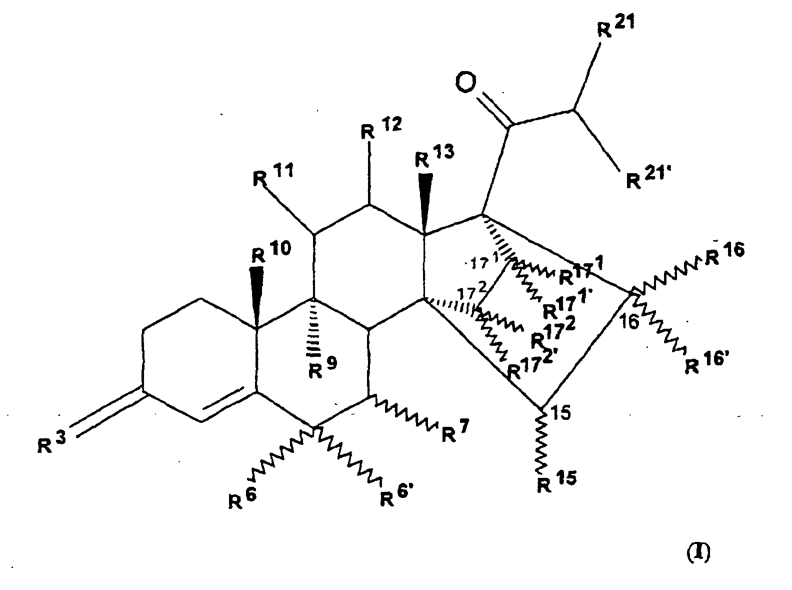 worin
R³ für ein Sauerstoffatom, die Hydroxyiminogruppe oder zwei Wasserstoffatome,
R⁶ für ein Wasserstoff-, Fluor-, Chlor- oder Bromatom oder für einen α- oder β-ständigen C₁-C₄-Alkylrest, wobei dann R⁶' und R⁷ Wasserstoffatome darstellen, oder aber
R⁶' für ein Wasserstoff-, Fluor-, Chlor- oder Bromatom oder für einen C₁-C₄-Alkylrest, wobei dann R⁶' und R⁷ eine gemeinsame zusätzliche Bindung darstellen,
R⁷ für einen α- oder β-ständigen C₁-C₄-Alkylrest, wobei dann R⁶ und R⁶' Wasserstoffatome darstellen, oder aber
R⁶ und R⁷ gemeinsam für eine α- oder β-ständige Methylengruppe und R⁶' für ein Wasserstoffatom oder
R⁶ und R⁶' gemeinsam für eine Ethylen- oder Methylengruppe und R⁷ für ein Wasserstoffatom,
R⁹ und R¹⁰ jeweils für ein Wasserstoffatom oder eine gemeinsame Bindung,
R¹¹ und R¹² jeweils für ein Wasserstoffatom oder eine gemeinsame Bindung,
R¹³ für eine Methyl- oder Ethylgruppe.
R¹⁵ für ein Wasserstoffatom oder einen C₁-C₃-Alkylrest,
R¹⁶ und R¹⁶' unabhängig voneinander für ein Wasserstoffatom, einen C₁-C₃-Alkylrest oder einen C₂-C₄-Alkenylrest oder gemeinsam für eine C₁-C₃-Alkylidengruppe
R¹⁵ und R¹⁶ für eine gemeinsame Bindung sowie R^{16'} für ein Wasserstoffatom oder einen C₁-C₃-Alkylrest oder
R¹⁵ und R¹⁶ gemeinsam für einen Ring der Teilformel 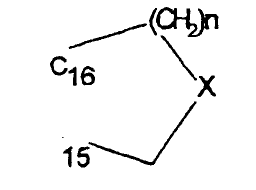
worin n = 1 und 2 und X eine Methylengruppe oder ein Sauerstoffatom bedeuten sowie R¹⁶' für ein Wasserstoffatom,
R^{17¹} für ein Wasserstoffatom oder einen C₁-C₃-Alkylrest,
R^{17²} für ein Wasserstoffatom, einen C₁-C₃-Alkylrest oder einen C₂-C₄-Alkenylrest
R^{17^{1'}} und R^{17^{2'}} jeweils für ein Wasserstoffatom oder für eine gemeinsame Bindung,
R²¹ für ein Wasserstoffatom oder einen C₁-C₃-Alkylrest,
R²¹' für eine Hydroxygruppe stehen.

2. Kombination nach Anspruch 1, wobei das Gestagen ein (21S)-21-Hydroxy-21-methyl-14,17-ethano-19-norpregna-4,9,15-triene-3,20-dion ist.

3. Kombination nach einem der vorherigen Ansprüche, wobei das Cyclodextrin ein β-Cyclodextrin ist.

4. Kombination nach einem der vorherigen Ansprüche, wobei das Cyclodextrin und das Gestagen
beim β-Cyclodextrin in einem Komplex 1 : n (Gestagen : Cyclodextrin, n≥ 1) vorliegt, und
beim γ-Cyclodextrin in einem Komplex 1 : n (n ≥ 1) (Gestagen : Cyclodextrin) vorliegt.

5. Kombination nach einem der vorherigen Ansprüche als Arzneimittel.

6. Kombination nach Anspruch 5 als stabile orale Formulierung.

7. Kombination nach Anspruch 5 oder 6 zur Herstellung eines Arzneimittels zur Behandlung von klimakterischen Beschwerden.

8. Kombination nach einem der vorherigen Ansprüche 1 bis 4 für die Fertilitätskontrolle.

9. Arzneimittel oder pharmazeutisches Präparat enthaltend eine Kombination nach einem der vorherigen Ansprüche mit pharmazeutisch verträglichen Hilfsstoffen und Trägerstoffen.

10. Arzneimittel oder pharmazeutisches Präparat enthaltend eine Kombination nach einem der vorherigen Ansprüche zur peroralen, oralen, parenteralen, transdermalen, pulmonalen, nasalen, rektalen, vaginalen oder intrauterinen Anwendung.

11. Verwendung einer Kombination nach einem der vorherigen Ansprüche 1 bis 8 zur Herstellung eines Medikaments zur Behandlung prämenstrueller Beschwerden, wie Kopfschmerzen, depressive Verstimmung, Wasserretention und Mastodynie.

12. Verfahren zur Stabilisierung eines Gestagens nach der Formel I gemäß Anspruch 1 unter Verwendung von einem β-Cyclodextrin oder γ-Cyclodextrin oder Derivaten dieser Cyclodextrine, die durch Veretherung oder Veresterung freier alkoholischer Funktionen der Cyclodextrine erhalten werden.

13. Verfahren zum Komplexieren von einem Gestagen nach Anspruch 1 und von einem β-Cyclodextrin oder γ-Cyclodextrin unter Verreiben als Trockenmischung oder durch Fällungsreaktion, vorzugsweise Copräzipitation.

14. Verfahren der Direkttablettierung von einem Gestagenkomplex nach Anspruch 1 mit einem β-Cyclodextrin oder γ-Cyclodextrin unter Zugabe von pharmazeutisch verträglichen Hilfsstoffen.

## Claims

1. Combination of at least one progestogen and one β-cyclodextrin or γ-cyclodextrin or derivatives of these cyclodextrins which are obtained by etherification or esterification of free alcoholic functions of the cyclodextrins, where the progestogens belong to the group of the formula I: 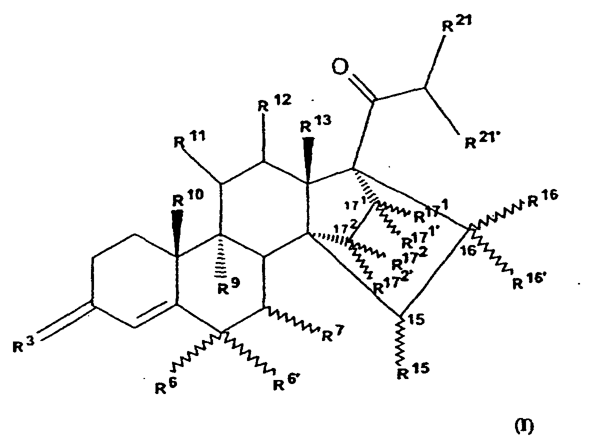 in which
R³ is an oxygen atom, the hydroxyimino group or two hydrogen atoms,
R⁶ is a hydrogen, fluorine, chlorine or bromine atom or is an α- or β-C₁-C₄-alkyl radical, in which case R⁶' and R⁷ are hydrogen atoms, or else
R⁶' is a hydrogen, fluorine, chlorine or bromine atom or is a C₁-C₄-alkyl radical, in which case R⁶' and R⁷ are a mutual additional bond,
R⁷ is an α- or β-C₁-C₄-alkyl radical, in which case R⁶ and R⁶' are hydrogen atoms, or else
R⁶ and R⁷ together are an α- or β-methylene group and R⁶' is a hydrogen atom or
R⁶ and R⁶' together are an ethylene or methylene group and R⁷ is a hydrogen atom,
R⁹ and R¹⁰ are each a hydrogen atom or a mutual bond,
R¹¹ and R¹² are each a hydrogen atom or a mutual bond,
R¹³ is a methyl or ethyl group,
R¹⁵ is a hydrogen atom or a C₁-C₃-alkyl radical,
R¹⁶ and R¹⁶' are, independently of one another, a hydrogen atom, a C₁-C₃-alkyl radical, or a C₂-C₄-alkenyl radical or together are a C₁-C₃-alkylidene group
R¹⁵ and R¹⁶ are a mutual bond, and R¹⁶' is a hydrogen atom or a C₁-C₃-alkyl radical or
R¹⁵ and R¹⁶ together are a ring of the part-formula 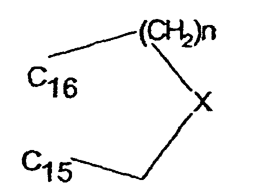
in which n = 1 and 2 and X is a methylene group or an oxygen atom, and R¹⁶' is a hydrogen atom,
R^{17¹} is a hydrogen atom or a C₁-C₃-alkyl radical,
R^{17²} is a hydrogen atom, a C₁-C₃-alkyl radical or a C₂-C₄-alkenyl radical
R^{17¹} and R^{17²} are each a hydrogen atom or a mutual bond,
R²¹ is a hydrogen atom or a C₁-C₃-alkyl radical,
R²¹' is a hydroxyl group.

2. Combination according to Claim 1, where the progestogen is a (21S)-21-hydroxy-21-methyl-14,17-ethano-19-norpregna-4,9,15-triene-3,20-dione.

3. Combination according to either of the preceding claims, where the cyclodextrin is a β-cyclodextrin.

4. Combination according to any of the preceding claims, where the cyclodextrin and the progestogen
is present in the case of β-cyclodextrin in a 1:n (progestogen:cyclodextrin n ≥ 1) complex, and
is present in the case of γ-cyclodextrin in a 1:n (n ≥ 1) (progestogen:cyclodextrin) complex.

5. Combination according to any of the preceding claims as medicament.

6. Combination according to Claim 5 as stable oral formulation.

7. Combination according to Claim 5 or 6 for producing a medicament for the treatment of climacteric symptoms.

8. Combination according to any of the preceding Claims 1 to 4 for fertility control.

9. Medicament or pharmaceutical product comprising a combination according to any of the preceding claims with pharmaceutically acceptable excipients and carriers.

10. Medicament or pharmaceutical product comprising a combination according to any of the preceding claims for peroral, oral, parenteral, transdermal, pulmonary, nasal, rectal, vaginal or intrauterine use.

11. Use of a combination according to any of the preceding Claims 1 to 8 for producing a medicament for the treatment of premenstrual symptoms such as headaches, depressive mood, water retention and mastodynia.

12. Method for stabilizing a progestogen according to formula I as set forth in Claim 1 using a β-cyclodextrin or γ-cyclodextrin or derivatives of these cyclodextrins which are obtained by etherification or esterification of free alcoholic functions of the cyclodextrins.

13. Method for complexation of a progestogen according to Claim 1 and of a β-cyclodextrin or γ-cyclodextrin by trituration as dry mixture or by precipitation reaction, preferably coprecipitation.

14. Method of direct tabletting of a progestogen complex according to Claim 1 with a β-cyclodextrin or γ-cyclodextrin with addition of pharmaceutically acceptable excipients.

## Revendications

1. Combinaison d'au moins un gestagène et d'une β-cyclodextrine ou γ-cyclodextrine ou des dérivés de ces cyclodextrines, obtenus par éthérification ou estérification des fonctions alcooliques libres des cyclodextrines, les gestagènes appartenant au groupe de formule I 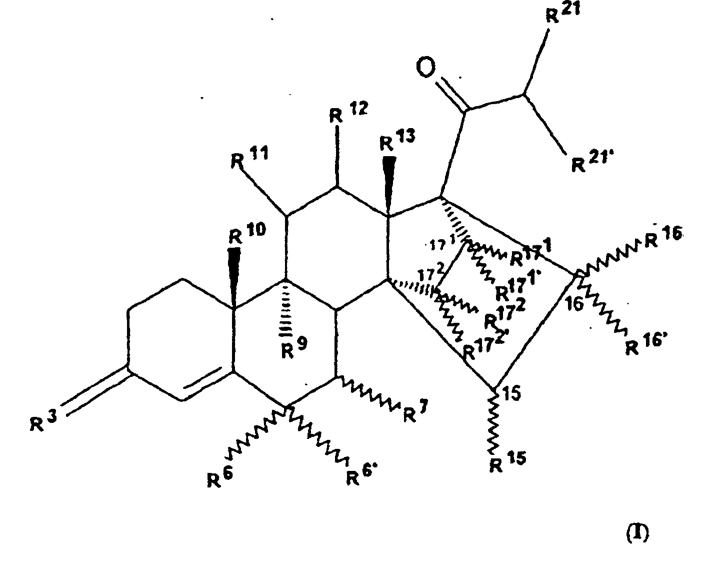 dans laquelle
R³ représente un atome d'oxygène, le groupe hydroxyimino ou deux atomes d'hydrogène,
R⁶ représente un atome d'hydrogène, de fluor, de chlore ou de brome ou un radical alkyle en C₁ à C₄ en position α ou β, R⁶' et R⁷ représentant alors des atomes d'hydrogène, ou
R⁶' représente un atome d'hydrogène, de fluor, de chlore ou de brome ou un radical alkyle en C₁ à C₄, R⁶' et R⁷ représentant alors une liaison commune supplémentaire,
R⁷ représente un radical alkyle en C₁ à C₄ en position α ou β, R⁶ et R⁶' représentant alors des atomes d'hydrogène ou
R⁶ et R⁷ représentent ensemble un groupe méthylène en position α ou β et R⁶' représente un atome d'hydrogène ou
R⁶ et R⁶' représentent ensemble un groupe éthylène ou méthylène et R⁷ représente un atome d'hydrogène,
R⁹ et R¹⁰ représentent à chaque fois un atome d'hydrogène ou une liaison commune,
R¹¹ et R¹² représentent à chaque fois un atome d'hydrogène ou une liaison commune,
R¹³ représente un groupe méthyle ou éthyle,
R¹⁵ représente un atome d'hydrogène ou un radical alkyle en C₁ à C₃,
R¹⁶ et R¹⁶' représentent, indépendamment l'un de l'autre, un atome d'hydrogène, un radical alkyle en C₁ à C₃ ou un radical alcényle en C₂ à C₄ ou, ensemble, un groupe alkylidène en C₁ à C₃,
R¹⁵ et R¹⁶ représentent une liaison commune et R¹⁶' représente un atome d'hydrogène ou un radical alkyle en C₁ à C₃ ou
R¹⁵ et R¹⁶ représentent ensemble un cycle de formule partielle 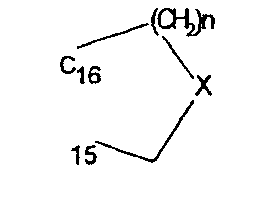
dans laquelle n = 1 et 2 et X représente un groupe méthylène ou un atome d'oxygène et R¹⁶' représente un atome d'hydrogène,
R^{17¹} représente un atome d'hydrogène ou un radical alkyle en C₁ à C₃,
R^{17²} représente un atome d'hydrogène, un radical alkyle en C₁ à C₃ ou un radical alcényle en C₂ à C₄,
R^{17^{1'}} et R^{17^{2'}} représentent à chaque fois un atome d'hydrogène ou une liaison commune,
R²¹ représente un atome d'hydrogène ou un radical alkyle en C₁ à C₃,
R²¹' représente un groupe hydroxy.

2. Combinaison selon la revendication 1, le gestagène étant une (21S)-21-hydroxy-21-méthyl-14,17-éthano-19-norpregna-4,9,15-triène-3,20-dione.

3. Combinaison selon l'une quelconque des revendications précédentes, la cyclodextrine étant une β-cyclodextrine.

4. Combinaison selon l'une quelconque des revendications précédentes, la cyclodextrine et le gestagène se trouvant
dans un complexe 1:n (gestagène:cyclodextrine, n≥1) dans le cas de la β-cyclodextrine
dans un complexe 1:n (n≥1) (gestagène:cyclodextrine) dans le cas de la γ-cyclodextrine.

5. Combinaison selon l'une quelconque des revendications précédentes en tant que médicament.

6. Combinaison selon la revendication 5 en tant que formulation orale stable.

7. Combinaison selon la revendication 5 ou 6 pour la préparation d'un médicament pour le traitement de troubles de la ménopause.

8. Combinaison selon l'une quelconque des revendications précédentes 1 à 4 pour le contrôle de la fertilité.

9. Médicament ou préparation pharmaceutique contenant une combinaison selon l'une quelconque des revendications précédentes avec des adjuvants et des supports pharmaceutiquement acceptables.

10. Médicament ou préparation pharmaceutique contenant une combinaison selon l'une quelconque des revendications précédentes pour l'administration perorale, orale, parentérale, transdermique, pulmonaire, nasale, rectale, vaginale ou intra-utérine.

11. Utilisation d'une combinaison selon l'une quelconque des revendications précédentes 1 à 8 pour la préparation d'un médicament pour le traitement de troubles prémenstruels, tels que les céphalées, l'humeur dépressive, la rétention d'eau et la mastodynie.

12. Procédé pour la stabilisation d'un gestagène de formule I selon la revendication 1 avec utilisation d'une β-cyclodextrine ou d'une γ-cyclodextrine ou des dérivés de ces cyclodextrines qui sont obtenus par éthérification ou estérification des fonctions alcooliques libres des cyclodextrines.

13. Procédé pour la complexation d'un gestagène selon la revendication 1 et d'une β-cyclodextrine ou d'une γ-cyclodextrine par broyage sous forme d'un mélange sec ou par une réaction de précipitation, de préférence une coprécipitation.

14. Procédé pour la transformation directe en comprimés d'un complexe de gestagène selon la revendication 1 avec une β-cyclodextrine ou une γ-cyclodextrine avec addition d'adjuvants pharmaceutiquement acceptables.
